# FASCICULE DE BREVET EUROPEEN

(11) **EP 1 423 707 B1**
(45) Date de publication et mention de la délivrance du brevet: **07.11.2007**
(21) Numéro de dépôt: 02781372.4
(22) Date de dépôt: 06.09.2002
(51) Int. Cl.: G01N 33/92

(54) **METHODES DE CRIBLAGE DE MOLECULES UTILES POUR LA PREVENTION DES MALADIES CARDIOVASCULAIRES**
VERFAHREN ZUM AUFFINDEN VON VERBINDUNGEN, DIE DER VORSORGE GEGEN CARDIOVASCULÄRE KRANKHEITEN DIENEN
METHODS OF SCREENING MOLECULES THAT ARE USED TO PREVENT CARDIOVASCULAR DISEASES

(30) Priorité: 07.09.2001 FR 0111598
(43) Date de publication de la demande: 02.06.2004
(73) Titulaire: Genfit, 59120 Loos (FR)
(72) Inventeur: NAJIB, Jamila, 40000 Marrakech (MA); MAJD, Zouher, F-59320 Ennetieres en Weppes (FR)
(74) Mandataire: Tezier Herman, Béatrice
(86) Numéro de dépôt international: PCT/FR2002/003040
(87) Numéro de publication internationale: WO 2003/023407

(56) Documents cités:
- WO-A-00/03013
- WO-A-00/37491
- WO-A-01/00803
- WO-A-01/79446

## Description

La présente invention concerne des compositions et méthodes de criblage de molécules utiles pour la prévention ou le traitement du syndrome métabolique, des maladies cardiovasculaires et/ou de l'athérosclérose. L'invention concerne notamment des méthodes de criblage de composés basées sur la détermination de l'effet de composés tests sur l'activité d'une nouvelle protéine apparentée à l'apolipoprotéine AIV (AA4RP). L'invention concerne également des compositions et méthodes pour diminuer la concentration de triglycérides et/ou pour diminuer la concentration ou l'expression de l'apolipoprotéine CIII (apo CIII) et/ou pour augmenter l'activité de la lipase hépatique (HL) et de la lipoprotéine lipase (LpL) et/ou pour diminuer la concentration de VLDL.

Le syndrome métabolique et les maladies cardiovasculaires sont devenues les maladies les plus préoccupantes au niveau mondial. L'athérosclérose qui les accompagne et qui est à l'origine de nombreux décès (infarctus du myocarde, embolie cérébrale, etc.) est une maladie multifactorielle.

En raison de la découverte des statines et de la mise en oeuvre de nombreux essais d'intervention avec ces molécules, tous positifs, l'attention a été focalisée ces dix dernières années sur les LDL (Low Density Lipoproteins), le LDL cholestérol étant choisi comme le paramètre majeur des autorités puisqu'il a été démontré une relation directe entre la concentration des LDL et le risque de maladies cardiovasculaires.

Aujourd'hui, la publication de nombreuses études épidémiologiques et d'un essai (VAHIT) démontre qu'à côté des LDL, les triglycérides et les HDL (High Density Lipoproteins,) jouent un rôle clef dans l'athérogénèse. Les HDL sont des lipoprotéines anti-athérogènes, leur concentration est corrélée de façon inverse au risque. Le rôle des triglycérides, et en particulier des lipoprotéines riches en triglycérides et/ou contenant l'apolipoprotéine CIII (apo CIII), en tant que facteur de risque indépendant pour l'athérosclérose n'est plus discuté.

L'apo CIII est une protéine de 79 acides aminés synthétisée par le foie et l'intestin [1]. C'est un composant des chylomicrons, des VLDL (very low density lipoproteins) et des HDL (high density lipoproteins) [2].

La concentration de d'apo CIII joue un rôle important dans le contrôle du métabolisme des triglycérides du plasma et dans la détermination de la concentration plasmatique de lipoprotéines riches en triglycérides potentiellement athérogéniques [3], puisqu'il inhibe leur lipolyse en bloquant l'activité de la LpL .

La concentration en apo CIII peut être corrélée avec de multiples états pathophysiologiques impliqués dans la prédisposition à l'athérosclérose et aux maladies cardiovasculaires.

Il a été également démontré que la concentration en apo CIII dans les particules VLDL et LDL est une mesure plus spécifique des risque de maladies cardiovasculaires que la concentration en triglycérides plasmatiques [4].

La concentration des triglycérides est également contrôlée par l'action lipolytique de la LpL et de la HL. *In vivo,* les substrats préférés de la LpL sont les larges particules représentées par les lipoprotéines riches en triglycérides tels que les chylomicrons et les VLDL, alors que la HL est plus efficace dans l'hydrolyse des lipoprotéines de plus petites taille comme les IDL et les HDL [5, 6](en particulier les HDL₂ [7]).

La LpL et la HL jouent également un rôle dans le transport inverse du cholestérol grâce à leur action sur le remodelage des HDL. La LpL par son action sur les lipoprotéines riches en triglycérides participe à la formation des préβ-HDL qui sont les premiers accepteurs du cholestérol cellulaire [8-10].

La HL comme la LpL participe à la formation des préβ-HDL mais le mécanisme d'action est différent. La HL hydrolyse les HDL₂ riches en triglycérides et le résultats de cette hydrolyse est la formation de petites HDL de type préβ-HDL participant ainsi à la captation sélective du cholestérol HDL par le foie [11]. Des travaux ont montré que la HL et le SR-BI (récepteur hépatique impliqué dans la captation des HDL et le transfert de leur cholestérol dans le foie) ont une action combinée et non parallèle pour promouvoir l'efflux du cholestérol [7], montrant ainsi le rôle essentiel de l'enzyme dans le phénomène du transport inverse du cholestérol.

La découverte ou la mise au point de molécules, de méthodes ou de kits pour moduler, et notamment pour réduire la quantité d'apo CIII est donc une avancée majeure dans le domaine thérapeutique, diagnostic et/ou expérimental.

La découverte ou la mise au point de molécules, de méthodes ou de kits pour moduler, et notamment pour réduire la quantité de triglycérides ou de VLDL ou pour réguler le transport inverse du cholestérol est donc également une avancée majeure dans le domaine thérapeutique, diagnostic et/ou expérimental.

La présente invention propose une solution alternative aux stratégies thérapeutiques de l'art antérieur pour la prise en charge du syndrome métabolique et des pathologies cardiovasculaires. La présente invention décrit en effet la caractérisation fonctionnelle d'une nouvelle protéine qui représente une cible particulièrement avantageuse pour la recherche et le développement de molécules actives. En outre, cette cible présente l'avantage important d'être spécifique des voies métaboliques considérées.

Plus particulièrement, la présente demande résulte de l'identification d'une nouvelle protéine régulant le métabolisme des lipides et des lipoprotéines, notamment des lipoprotéines riches en triglycérides et en apolipoprotéine CIII (Apo CIII).

Cette protéine présente la structure primaire représentée ci-dessous, comprenant 366 acides aminés (SEQ ID NO 1).

Cette protéine correspond à la séquence de la protéine RAP3 mentionnée antérieurement comme potentiellement impliquée dans la régénération hépatique (numéro d'accès Genbank: AF202889.1 : Van der Vliet H.N., Groenink M., Leegwater A.C.J. and Chamuleau R.A.F.M. Submitted (09-NOV-1999) Experimental Hepatology, Academic Medical Center, Meibergdreef 9, Amsterdam 1105 AZ, Netherlands).

Elle correspond également à la protéine AA4RP (« Apolipoprotein AIV-Related Protein ») de la demande de brevet WO01/00803 A2 (SEQ ID NO 3 de cette demande) déposée au nom de la société Genset. Cette protéine AA4RP est un membre de la famille des apolipoprotéines impliquée dans le métabolisme des lipides.

La présente demande montre à présent que la protéine ci-dessus désignée AA4RP est capable de diminuer in vivo la concentration en triglycérides et en VLDL. La demande montre que cette protéine permet aussi de diminuer in vivo la concentration en apo CIII, marqueur des lipoprotéines athérogènes.

La présente demande résulte donc de la découverte du rôle fonctionnel d'une nouvelle protéine, désignée AA4RP dans la régulation du métabolisme des lipoprotéines, notamment celles contenant les triglycérides et l'apo CIII.

La présente demande décrit également la production de peptides dérivés de la structure primaire de l'AA4RP, comprenant un ou des domaines immunogènes de l'AA4RP.

La présente demande décrit encore la fabrication, à partir de peptides immunogènes, d'anticorps reconnaissant l'AA4RP dans les lipoprotéines humaines, ou éventuellement sous forme libre, et permettant ainsi son dosage.

Le développement de cet anticorps anti-AA4RP a permis de localiser cette apolipoprotéine au niveau des HDL, confirmant son rôle très probable dans le transport inverse du cholestérol.

L'invention décrit ainsi une nouvelle cible et des outils utilisables pour la mise au point de nouvelles molécules actives pour prévenir ou traiter le syndrome métabolique, les maladies cardiovasculaire, et en particulier l'athérosclérose. L'invention est également utilisable pour la détection d'une prédisposition ou d'un risque de développement du syndrome métabolique, de pathologies cardiovasculaires et en particulier de l'athérosclérose.

Un premier objet de l'invention réside donc dans l'utilisation de l'AA4RP pour le développement de composés actifs sur les pathologies cardiovasculaires ou métaboliques.

### Protéine apparentée à l'apolipoprotéine AIV (AA4RP)

Dans le contexte de l'invention, le terme AA4RP (ou protéine AA4RP) désigne un polypeptide comprenant la séquence SEQ ID NO 1 ou tous variants, dérivés ou homologues. Il s'agit plus particulièrement de tout variant naturel de la séquence SEQ ID NO 1, résultant de polymorphisme, épissage, mutations, etc.. De tels variants naturels peuvent donc comprendre une ou plusieurs mutations
ou substitutions, une délétion d'un ou plusieurs résidus, etc.. Le terme homologue désigne par ailleurs les polypeptides AA4RP d'autres espèces, par exemple de rongeurs, bovins, etc..

De manière générale, le terme AA4RP désigne un polypeptide de séquence SEQ ID NO 1 ou tout dérivé comprenant une ou plusieurs mutations, délétions, substitutions ou additions d'un ou plusieurs acides aminés. De préférence, il s'agit de polypeptides reconnus par un anticorps polyclonal produit à partir de l'AA4RP de séquence SEQ ID NO 1. Encore plus préférentiellement, des analogues sont des polypeptides conservant au moins une propriété immunologique ou biologique de lAA4RP. Les propriétés biologiques sont notamment la régulation de la concentration des triglycérides ou de l'apo CIII, par exemple. Des variants préférés comportent au moins 80% de la séquence SEQ ID NO 1, plus préférentiellement au moins 90% d'identité avec la séquence SEQ ID NO 1. Le degré d'identité peut être déterminé par exemple selon la méthode CLUSTAL. Des variants particuliers possèdent une mutation ou une substitution affectant 5 acides aminés au plus de la séquence SEQ ID NO 1.

Des dérivés particuliers selon l'invention sont des fragments de l'AA4RP ci-dessus, notamment des polypeptides comprenant une partie de la séquence SEQ ID NO 1. A cet égard, un objet de l'invention réside dans un polypeptide caractérisé en ce qu'il consiste en la sequence des résidus 20-114.

La présente demande décrit en effet la production de polypeptides fragments de l'AA4RP, comportant de préférence une portion immunogène de l'AA4RP, qui sont utilisables pour la production d'anticorps, de tests de dosages, comme compétiteurs, etc..

Ces polypeptides ont été élaborés en utilisant notamment des algorithmes permettant d'évaluer la flexibilité [12, 13], l'hydrophilicité [14, 15], l'antigénicité [16] et les structures secondaires [17, 18].

De cette manière, les demandeurs ont identifié des régions intéressantes sur le plan immunogène ou biologique de la molécule. A titre d'exemple particulier, on peut citer un polypeptide correspondant aux (ou comprenant les) résidus 20-114 de la séquence SEQ ID NO 1.

Les polypeptides fragments de la présente divulgation contiennent de préférence moins de 200 acides aminés, plus préférentiellement moins de 150 acides aminés. Ils contiennent de préférence au moins un épitope de l'AA4RP. Des polypeptides préférés de l'invention comprennent moins de 200 acides aminés de la séquence SEQ ID NO 1 et comportent au moins les résidus 50-80 de la séquence SEQ ID NO 1.

Au sens de la présente divulgation terme "fragment immunogène" désigne toute portion X du polypeptide comprenant un épitope, de préférence un épitope T ou B. Un tel fragment comprend donc avantageusement au moins 7 acides aminés consécutifs de la séquence SEQ ID NO 1 , plus préférentiellement au moins 10 acides aminés consécutifs de la séquence SEQ ID NO 1, encore plus préférentiellement au moins 15 acides aminés consécutifs de la séquence SEQ ID NO 1.

Les polypeptides de la présente divulgation peuvent comprendre des résidus hétérologues ajoutés à la séquence d'acides amines indiquée. Ainsi, un objet de l'invention réside dans un polypeptide comprenant tout ou partie de la séquence SEQ ID NO 1, ou d'un variant naturel de celle-ci, et une partie hétérologue.

La partie hétérologue peut correspondre à des acides aminés, lipides, sucres, etc.. Il peut également s'agir de groupe(s) chimique(s), enzymatique(s), radioactif(s), etc.. L'élément hétérologue peut en particulier constituer un marqueur, un agent de ciblage, un agent stabilisateur, un agent améliorant l'immunogénicité ou facilitant la production, un agent protecteur, un agent facilitant la pénétration du peptide dans les cellules, une toxine, ou composé actif, un anticorps, etc..

Les polypeptides de l'invention peuvent se présenter sous forme soluble, purifiée ou complexée avec une molécule porteuse telle que KLH ou la sérum-albumine, ou toute autre molécule inerte (par exemple synthétique), telle qu'une bille par exemple. Les polypeptides selon l'invention sont préférentiellement dépourvus de contamination par des protéines naturellement présentes dans le sang, notamment par une autre apolipoprotéine. Dans un mode de mise en oeuvre particulier, les polypeptides sont couplés à une molécule porteuse notamment pour la fabrication d'anticorps. Le couplage peut être réalisé selon des techniques conventionnelles [19, 20]. Les polypeptides peuvent également être conjugués ou fusionnés à toute autre molécule polypeptidique ou peptidique telle que par exemple un peptide, polypeptide ou une protéine biologiquement active.

Les polypeptides peuvent être préparés par synthèse artificielle selon des techniques conventionnelles telles que la synthèse en phase solide décrite par [21] ainsi que dans les exemples. La synthèse artificielle est avantageuse dans la mesure ou les polypeptides produits sont dépourvus de toute contamination par des produits d'origine naturelle notamment par des dérivés sanguins. Il est entendu que les polypeptides de l'invention peuvent être fabriqués par toute technique biologique, génétique ou enzymatique, et notamment par expression dans un hôte cellulaire approprié d'un acide nucléique correspondant. A cet égard, un autre objet de la présente invention réside dans une molécule d'acide nucléique codant un polypeptide tel que défini ci-dessus. Cet acide nucléique peut être un ADN ou un ARN, de préférence un ADNc, et peut éventuellement comprendre une région promoteur. L'acide nucléique peut en outre être inséré dans un vecteur (par exemple un plasmide, un cosmide, un phage, un virus, un chromosome artificiel, etc.). De tels acides nucléiques peuvent être utilisés pour produire des polypeptides selon l'invention in vitro ou directement in vivo.

Les polypeptides selon l'invention peuvent être utilisés dans des tests de screening ou de criblage, dans des tests de titration ou comme standard par exemple pour calibrer les essais. Ils peuvent également être utilisés pour réguler l'activité de l'AA4RP in vitro ou in vivo. Ils sont particulièrement intéressants pour la production d'anticorps anti-AA4RP.

Un objet de l'invention réside dans une composition comprenant un polypeptide tel que défini dans la revendication 5. Une composition particulière comprend le polypeptide et un véhicule acceptable sur le plan pharmaceutique. Le véhicule peut être une solution saline, isotonique, tamponnée, éventuellement combinée à un agent stabilisant, émulsifiant, etc..

### Anticorps

Un autre objet de la présente divulgation réside dans un anticorps reconnaissant un polypeptide tel que défini ci-dessus. Il s'agit avantageusement d'un anticorps spécifique d'un tel polypeptide, c'est-à-dire préparé par immunisation avec un tel polypeptide.

L'anticorps peut être polyclonal ou monoclonal. D'autre part, le terme anticorps désigne également tous fragments ou dérivés d'anticorps en particulier des fragments ou dérivés d'anticorps monoclonaux ou polyclonaux conservant la même spécificité antigénique. De tels fragments ou dérivés d'anticorps comprennent par exemple les fragments Fab, Fab'2, CDRs, etc., des anticorps humanisés, des anticorps polyfonctionnels, des anticorps simple chaîne (ScFv), etc.. Ces anticorps peuvent être produits selon des techniques conventionnelles comprenant l'immunisation d'un animal et la récupération du sérum (polyclonal) ou des cellules de la rate (pour produire des hybridomes par fusion avec des lignées cellulaires appropriées).

Des méthodes de production d'anticorps polyclonaux à partir d'espèces diverses telles que souris, rongeurs, primates, chevaux, cochons, lapins, volailles, etc. peuvent être trouvées dans [19]. De manière brève, l'antigène est combiné à un adjuvant, par exemple l'adjuvant de Freud, et est administré à un animal, classiquement par injection sous cutanée. Des injections répétées peuvent être réalisées. Des échantillons de sang sont récoltés et les immunoglobulines ou le sérum sont séparés.

Des méthodes pour la production d'anticorps monoclonaux peuvent être trouvées notamment dans Harlow et al. [22] ou dans [23]. De manière brève, ces méthodes comprennent l'immunisation d'un animal avec un antigène suivi de la récupération des cellules de la rate qui sont ensuite fusionnées avec des cellules immortalisées telles que des cellules de myélome. Les hybridomes ainsi obtenus produisent les anticorps monoclonaux et peuvent être sélectionnés par dilution limite pour isoler les clones individuels. Les anticorps peuvent aussi être produits par sélection de banques combinatoires d'immunoglobulines, tel que décrit par exemple dans [24].

Des fragments Fab ou Fab'2 peuvent être produits par digestion avec des protéases selon des techniques conventionnelles. Des anticorps humanisés peuvent être préparés tels que décrits par exemple par [25, 26].

La présente divulgation concerne également un anticorps dirigé contre un épitope compris dans les résidus 20-114 de la séquence SEQ ID NO 1.

La présente divulgation concerne également tout anticorps anti-AA4RP, caractérisé en ce qu'il est obtenu par immunisation d'un mammifère non-humain avec un peptide immunogène comprenant une partie de la séquence SEQ ID NO 1. Un tel anticorps polyclonal a été généré chez le lapin contre un peptide de synthèse de 95 acides aminés. Cet anticorps a permis de montrer, par western blot, que l'AA4RP est localisée essentiellement au niveau des HDL. Chez la souris contrôle, la sous fraction reconnue après immunoélectrophorèse bidimensionnelle se trouve au niveau des pré-β HDL qui sont les premiers accepteurs du cholestérol tissulaire au cours du transport inverse du cholestérol.

La présente divulgation concerne également une méthode de production d'un anticorps anti-AA4RP comprenant l'injection d'un polypeptide tel que défini ci-dessus, notamment d'un polypeptide comprenant toute ou partie de la séquence SEQ ID NO 1, notamment les résidus 50 à 80, à un mammifère non-humain, puis la récupération des anticorps ou des cellules productrices d'anticorps.

Les anticorps selon la divulgation peuvent être couplés à des parties hétérologues telles que des toxines, des marqueurs, des drogues ou d'autres agents thérapeutiques. Le couplage peut être covalent ou non, direct ou par l'intermédiaire d'un agent de couplage ou d'un espaceur. Les marqueurs peuvent être par exemple des marqueurs radioactifs, des enzymes, des agents fluorescents, des particules magnétiques, etc.. Les toxines sont par exemple la toxine diphtérique, botulique, ricine, etc.. Les drogues ou agents thérapeutiques sont par exemple des lymphokines, des antibiotiques, des antisens, des facteurs de croissance, etc.. Des méthodes pour réaliser le couplage de telles régions hétérologues sont décrites par exemple dans le brevet américain US 4,277,149.

Les anticorps de la présente divulgation présentent des applications multiples notamment des applications thérapeutiques, diagnostiques, prophylactiques, ainsi que dans le domaine expérimental, par exemple pour la purification d'antigènes. In vitro, ils peuvent être utilisés notamment comme agent de screening ou pour purifier l'antigène à partir d'échantillons biologiques variés (échantillons de sang tel que le plasma, ou le sérum, ou un autre fluide biologique tel que les urines, le liquide interstitiel, etc.). Ils peuvent également être utilisés pour détecter ou quantifier la présence (ou la quantité) d'AA4RP dans un échantillon prélevé à partir d'un sujet ou sur une culture cellulaire, typiquement un échantillon de sang prélevé à partir d'un mammifère, notamment d'un sujet humain, comme il sera décrit dans la suite du texte.

### Tests de Criblage

Un autre objet particulier de l'invention réside dans un procédé pour la sélection, l'identification ou la caractérisation de composés, caractérisé en ce qu'il comprend la détermination de la capacité d'un composé test à augmenter, en particulier augmenter, l'activité de l'AA4RP comme décrit dans les revendications 1 à 4.

Ce procédé permet donc de sélectionner, d'identifier ou de caractériser des composés suceptibles de réduire le taux d'apolipoprotéine CIII circulante ou de lipoparticules riches en apolipoprotéine CIII et/ou d'augmenter l'activité de la LpL et/ou de la HL et/ou d'augmenter le transport inverse du cholestérol par détermination de la capacité d'un composé test à moduler (diminuer ou augmenter), en particulier augmenter, l'activité de l'AA4RP. On détermine plus particulièrement les composés capables d'augmenter de manière sélective l'activité de l'AA4RP, c'est-à-dire essentiellement sans affecter de manière directe l'activité d'une autre apolipoprotéine. Les procédés de l'invention sont particulièrement destinés à la sélection, l'identification ou la caractérisation de composés actifs pour le traitement de l'athérosclérose ou de maladies cardiovasculaires.

Au sens de la présente demande, le terme « activité de l'AA4RP » désigne notamment la synthèse de cette protéine (transcription, traduction, etc.), sa maturation, son export ou sa sécrétion extracellulaire, son interaction avec un récepteur, son incorporation dans des particules (notamment des HDL et les VLDL), sa dégradation, etc.. Le composé augmentant l'activité de l'AA4RP peut donc être un agent augmentant la synthèse de l'AA4RP, un agent augmentant le transport ou la sécrétion de l'AA4RP, un compétiteur de l'AA4RP, un agent mimant l'activité de l'AA4RP, etc..

Selon un premier mode préféré de mise en oeuvre, le procédé de la présente divulgation comprend la détermination de la capacité d'un composé test à augmenter la synthèse de l'AA4RP, c'est-à-dire notamment la transcription ou la traduction de son gène ou ARN.

Selon un autre mode préféré de mise en oeuvre, le procédé de la présente divulgation comprend la détermination de la capacité d'un composé test à augmenter la concentration de l'AA4RP dans les particules HDL.

Selon un autre mode préféré de mise en oeuvre, le procédé de la présente divulgation comprend la détermination de la capacité d'un composé test à augmenter la concentration de l'AA4RP dans les particules VLDL.

Selon un autre mode préféré de mise en oeuvre, le procédé de la présente divulgation comprend la détermination de la capacité d'un composé test à mimer l'action biologique de l'AA4RP. Comme illustré dans les exemples, cette action biologique implique notamment la réduction des concentrations de triglycérides ou d'apo CIII.

Selon un autre mode préféré de mise en oeuvre, le procédé de la présente divulgation comprend la détermination de la capacité d'un composé test à mimer l'action biologique de l'AA4RP. Comme illustré dans les exemples, cette action biologique correspond notamment à l'augmentation de l'activité de la LpL et/ou de la HL.

Le procédé de l'invention peut être mis en oeuvre de différentes façons, en tests *in vitro,* cellulaires ou acellulaires, ou *in vivo.* Il peut s'agir de tests de liaison, de tests fonctionnels, etc..

Un premier test est basé sur la mesure d'une interaction entre un composé et l'AA4RP. Dans ce mode de réalisation, le procédé comprend la mise en contact du composé test avec l'AA4RP ou un fragment de celle-ci et la détermination de la liaison du composé test à l'AA4RP ou au fragment.

Cet test peut être réalisé in vitro, dans tout dispositif approprié (tube, boite, flasque, etc.). Il peut éventuellement être réalisé avec l'un des partenaires immobilisé, par exemple l'AA4RP (colonne, bille, support, verre, filtre, membrane, etc.). La liaison du composé test peut être mise en évidence par toute technique connue, notamment par électrophorèse, migration sur gel, immunochimie, etc.. La liaison peut notamment être mise en évidence en effectuant la réaction en présence d'un ligand marqué de l'AA4RP et en mesurant le déplacement de la liaison du ligand marqué par le composé test. Le ligand marqué peut être par exemple un anticorps anti-AA4RP ou un fragment ou dérivé d'un tel anticorps.

Un autre type de test selon la présente divulgation est basé sur l'activité transcriptionnelle de l'AA4RP. Ce procédé comprend la mise en contact du composé test avec un acide nucléique comprenant tout ou une partie de la séquence du promoteur du gène de l'AA4RP et la détermination de la liaison du composé test à l'acide nucléique ou de la modulation de l'activité de l'acide nucléique par le composé test.

Pour la mise en oeuvre du test de liaison, il est possible d'utiliser un acide nucléique comprenant tout ou partie de la séquence du promoteur, et de déterminer in vitro la capacité d'un composé test de lier celle-ci. La partie de la séquence comporte de préférence au moins 10 nucléotides consécutifs de la séquence du promoteur, encore plus préférentiellement au moins 20 nucléotides consécutifs de la séquence du promoteur. Par ailleurs, il est possible de tester en parallèle plusieurs fragments de la séquence du promoteur.

Pour la mise en oeuvre du test transcriptionnel, on utilise avantageusement un système rapporteur comprenant tout ou partie du promoteur du gène AA4RP liée de manière opérationnelle à un gène rapporteur. A cet égard, un mode préféré de mise en oeuvre du procédé comprend la mise en contact du composé test avec une cellule comprenant un gène rapporteur sous le contrôle d'un promoteur transcriptionnel comprenant tout ou une partie de la séquence du promoteur du gène de l'AA4RP, et la détermination de l'effet du composé test sur l'expression du gène rapporteur. La séquence du promoteur du gène de l'AA4RP est représentée en SEQ ID NO :2.

Un autre mode de mise en oeuvre du procédé selon la présente divulgation comprend la mise en contact du composé test avec une cellule exprimant l'AA4RP ou un fragment de celle-ci et la détermination de l'effet du composé test sur l'expression ou la sécrétion de l'AA4RP ou du fragment par la cellule.

Les procédés de l'invention peuvent être mis en oeuvre avec différents types de cellules, de promoteur, de gènes rapporteurs, et dans différentes conditions, comme il est décrit ci-après.

### a) Cellule hôte

Certaines méthodes de criblage, décrites par l'invention, prévoient une étape de mise en contact du composé test avec des cellules hôtes, dans des conditions particulières qui permettent de déterminer l'expression dans lesdites cellules d'un gène rapporteur, ou l'expression de l'AA4RP, ou d'autres étapes de la synthèse de l'AA4RP, et d'obtenir ainsi une information concernant l'effet du composé test. Classiquement, l'effet du composé test est comparé au niveau d'expression (du gène rapporteur) ou à l'activité déterminée en l'absence dudit composé.

Les cellules utilisées peuvent être toute cellule cultivable en laboratoire. Dans un mode préféré de la présente divulgation, il s'agit de cellules de mammifères (hépatocytes, fibroblastes, cellules endothéliales, musculaires, etc.). De manière encore plus préférée, ces cellules sont d'origine humaine. Il peut s'agir de cultures primaires ou de lignées établies. Dans un autre mode de mise en oeuvre, il est possible également d'utiliser des cellules procaryotes (bactéries), des cellules de levure (Saccharomyces, Kluyveromyces, etc.), des cellules végétales, etc..

### b) Système rapporteur

Selon certains modes de réalisation, la présente divulgation met en oeuvre un système rapporteur comprenant un gène rapporteur placé sous le contrôle d'un promoteur particulier. Cette construction, ou toute cassette ou vecteur la contenant, est introduite dans des cellules hôtes, utilisables pour des tests cellulaires.

Ledit gène rapporteur peut être notamment tout gène dont le produit de transcription ou d'expression peut être détecté ou dosé dans des extraits biologiques. Il peut s'agir, par exemple, du gène codant pour l'AA4RP humaine lui-même, ou encore du gène codant pour la lucifèrase et plus particulièrement pour la luciférase de luciole ou pour celle de Renilla, pour la phosphatase alcaline sécrétée, la galactosidase, la lactamase, la Chloramphenicol acetyl transférase (CAT), l'hormone de croissance humaine (hGH), la β-glucuronidase (Gluc) et la Green fluorescent protein (GFP) etc.. Il est entendu que le terme « gène » désigne, au sens large, tout acide nucléique, notamment un ADNc, un ADNg, un ADN synthétique, un ARN, etc..

Le gène rapporteur, quel qu'il soit, est placé sous le contrôle d'un promoteur comprenant au moins une partie de la séquence du promoteur du gène AA4RP tel que défini ci-avant ou d'un variant fonctionnel de celle-ci. Cette séquence particulière peut être présente à raison d'une ou de plusieurs copies dans le promoteur (préférentiellement 1 à 10 et encore plus préférentiellement 1 à 6), en amont ou en aval ou en interne, dans la même orientation ou dans l'orientation opposée. Dans un mode préféré de mise en oeuvre de l'invention, le gène rapporteur est placé sous le contrôle d'un promoteur qui comprend la séquence complète du promoteur du gène AA4RP, notamment humain.

A cet égard, un objet de la présente divulgation concerne également un acide nucléique comprenant un gène rapporteur sous contrôle d'un promoteur comprenant tout ou partie de la séquence du promoteur du gène AA4RP, notamment humain. La séquence du promoteur du gène de l'AA4RP est représentée en SEQ ID NO :2. Les quatre derniers résidus de la séquence correspondent au début de l'exon 1. Les résidus 1027 à 1032 correspondent à la boite TATA. La partie de la séquence du promoteur comprend avantageusement au moins 10 nucléotides consécutifs de cette séquence, de préférence au moins 20, plus préférentiellement au moins 30, encore plus préférentiellement au moins 50. Le promoteur peut comprendre en outre des régions hétérologues, provenant d'autres gènes ou promoteurs, comme par exemple des signaux silenceurs ou enhanceurs, des séquences conférant un caractère régulable ou spécifique de tissu, etc.. Le promoteur peut être un promoteur hybride associant des régions d'autres promoteurs, par exemple du promoteur du gène de la thymidine kinase (TK) du virus de l'herpès, du promoteur immédiat du CMV, du promoteur PGK, du promoteur SV40, etc..

La construction peut être introduite dans tout vecteur approprié, tel qu'un plasmide, cosmide, phage, virus, etc.. La construction ou le vecteur peut être introduit dans une cellule hôte par toute méthode classique, telle que électroporation, précipitation au phosphate de calcium, liposomes, agents transfectants, etc.. Les cellules ou leur descendance peuvent être cultivées dans tout milieu approprié (DMEM, RPMI, etc.).

### c) Mise en contact

Les composés tests peuvent être mis au contact des cellules à différents moments, selon leur(s) effet(s), leur concentration, la nature des cellules et l'appréciation technique. Le contact peut être effectué sur tout support approprié et notamment sur une plaque, une boite, dans un tube ou une flasque. Généralement, la mise en contact est réalisée dans une plaque multi-puits ce qui permet de conduire, en parallèle, des essais nombreux et variés. Parmi les supports typiques on trouve des plaques de microtitration et plus particulièrement des plaques 96 ou 384 puits (ou plus), faciles à manipuler.

Selon le support et la nature du composé test, des quantités variables de cellules peuvent être utilisées lors de la mise en oeuvre des méthodes décrites. De manière classique entre 10³ et 10⁶ cellules sont mises en contact avec un type de composé test, dans un milieu de culture approprié, et de manière préférentielle entre 10⁴ et 10⁵ cellules. A titre d'exemples : dans une plaque de 96 puits, 10⁵ cellules peuvent être incubées dans chaque puit avec une quantité voulue d'un composé test ; dans une plaque à 384 puits, moins de 10⁵ cellules et typiquement entre 1x10⁴ et 4x10⁴ cellules sont généralement incubées dans chaque puit avec le composé test.

La quantité (ou la concentration) de composé test peut être ajustée par l'utilisateur selon le type de composé (sa toxicité, sa capacité de pénétration cellulaire, etc.), le nombre de cellules, la longueur de la période d'incubation, etc.. Généralement, les cellules sont exposées à des quantités de composés test qui varient de InM à 1mM. Il est bien sûr possible de tester d'autres concentrations sans dévier de la présente invention. Chaque composé peut de plus être testé en parallèle à différentes concentrations.

Différents adjuvants et/ou vecteurs et/ou produits facilitant la pénétration des composés dans les cellules tels que des liposomes, des lipides cationiques ou des polymères peuvent en outre être utilisés, si nécessaire.

Le contact est typiquement maintenu entre quelques minutes et plusieurs heures, généralement entre 1 et 48 heures. En particulier, lorsque le test comprend l'expression d'un gène rapporteur, les cellules et les divers réactifs doivent, de préférence, rester en contact suffisamment longtemps pour permettre la synthèse de novo du produit d'expression du gène rapporteur.

### d) Mesure de l'effet

La mesure ou la mise en évidence d'un effet du composé test peut être réalisée de différentes façons, selon le test utilisé.

Des méthodes de détection de la liaison in vitro ont été mentionnées ci-avant. Pour des test cellulaires impliquant la détection ou la mesure de l'expression d'un système rapporteur, plusieurs solutions sont envisageables.

Il peut s'agir d'une détermination de l'activité transcriptionnelle. A cette fin, l'ARN total est extrait des cellules en culture dans des conditions expérimentales d'une part et dans une situation témoin d'autre part. Cet ARN est dosé (ou utilisé comme sonde) pour analyser les changements dans l'expression du ou des gène(s) rapporteur(s).

Il peut également s'agir d'une révélation ou d'un dosage du produit d'expression du gène rapporteur. Cette révélation (ou ce dosage) peut être obtenue à l'aide de techniques variées dont la nature dépend du type de gène rapporteur utilisé. La mesure peut, par exemple, correspondre à une densité optique ou à une émission fluorescente dans le cas d'une utilisation comme gène rapporteur du gène codant pour la β-galactosidase ou la luciférase.

Dans un mode particulier, l'expression du gène rapporteur est mesurée à travers le niveau d'hydrolyse d'un substrat du produit d'expression du gène rapporteur. Par exemple, de nombreux substrats peuvent être utilisés pour évaluer l'expression de la β-lactamase. Il peut notamment s'agir de tout produit contenant un noyau β-lactame et dont l'hydrolyse peut être contrôlée. Les substrats préférés sont ceux spécifiques de la β-lactamase (i.e., ils ne sont généralement pas hydrolysés dans les cellules de mammifères en l'absence de β-lactamase), ceux qui ne sont pas toxiques à l'égard des cellules de mammifères et/ou dont le produit d'hydrolyse peut être contrôlé facilement, par exemple par des méthodes basées sur la fluorescence, la radioactivité, une activité enzymatique ou toute autre méthode de détection.

Le produit d'expression peut également être dosé par des techniques immunologiques ou immunoenzymatiques, comme par exemple au moyen d'un anticorps spécifique. Ce système est particulièrement adapté pour doser par exemple l'AA4RP synthétisée par une cellule traitée ou non traitée par un composé test.

D'une manière générale, la présence du produit du gène rapporteur (ou du produit d'hydrolyse du substrat) peut être déterminée par des méthodes classiques connues de l'homme du métier (fluorescence, radioactivité, D.O., luminescence, FRET (voir WO 0037077), SPA, biopuces, méthodes immunologiques, etc.). Généralement, on détermine l'activité d'un composé test dans une cellule et cet effet est comparé au niveau d'activité en l'absence de composé test ou à une valeur moyenne déterminée en l'absence de tout composé test.

Un test secondaire permettant de valider, chez l'animal, la sélection des composés, peut aussi être réalisé grâce à la détermination de la quantité d'HDL exprimées ou grâce à la détermination d'une variation significative du transport inverse du cholestérol au niveau de cellules traitées avec lesdits composés par comparaison avec des cellules non traitées. Il est également possible de mesurer le taux plasmatique de triglycérides et/ou d'apo CIII.

Les composés susceptibles d'être identifiés par le procédé de l'invention peuvent être des composés de nature, structure et origine variées, notamment des composés biologiques, des facteurs nucléaires, des cofacteurs, etc., des composés chimiques, synthétiques, etc.. Il peut s'agir également de banques, notamment de chimiothèques ou de banques de protéines, de peptides ou d'acides nucléiques, etc..

### Utilisation des composés

Compte tenu des propriétés fonctionnelles physiologiques de la cible utilisée, les composés capables de moduler, en particulier d'augmenter ou de mimer, l'activité de l'AA4RP peuvent être utilisés pour le traitement curatif ou préventif de pathologies cardiovasculaires ou du syndrome métabolique par diminution du taux d'apolipoprotéine CIII dans le sang d'un patient, en particulier de lipoparticules riches en apolipoprotéine CIII, ou par augmentation de l'activité de la LpL et/ou de la HL ou par augmentation du transport inverse du cholestérol.
Ces composés peuvent traiter des pathologies variées, notamment des pathologies cardiovasculaires, le syndrome métabolique et en particulier l'athérosclérose.

A cet égard, la présente divulgation concerne généralement l'utilisation d'un composé modulant, de préférence augmentant ou mimant, l'activité de l'AA4RP pour la préparation d'un médicament destiné au traitement curatif ou préventif de pathologies métaboliques ou cardiovasculaires. Il s'agit plus particulièrement de pathologies telles que l'athérosclérose (due à une dyslipoprotéinémies ou non), l'insulino-résistance, le diabète de type II, etc..

Un autre objet de la présente divulgation réside dans l'utilisation d'un composé augmentant ou mimant l'activité de l'AA4RP pour la préparation d'un médicament destiné à réduire le taux de triglycérides et/ou d'apo CIII dans le sang, en particulier pour réduire le taux de lipoparticules riches en triglycérides ou en apo CIII.

Un autre objet de la présente divulgation réside dans l'utilisation d'un composé augmentant ou mimant l'activité de l'AA4RP pour la préparation d'un médicament destiné à augmenter l'activité de la LpL et/ou de la HL, en particulier pour réduire le taux des triglycérides dans lipoprotéines.

Un autre objet de la présente divulgation porte sur l'utilisation d'un composé augmentant ou mimant l'activité de l'AA4RP pour la préparation d'un médicament destiné à réguler le transport inverse du cholestérol. En particulier, cette régulation se fait par l'augmentation de l'activité de la LpL et/ou la HL et/ou le SR-BI.

Dans le contexte de la présente divulgation, le terme « traitement » désigne le traitement préventif, curatif, palliatif, ainsi que la prise en charge des patients (réduction de la souffrance, amélioration de la durée de vie, amélioration de la qualité de vie, ralentissement de la progression de la maladie), etc.. Le traitement peut en outre être réalisé en combinaison avec d'autres agents ou traitements, notamment adressant les événements tardifs de la pathologie ou d'autres principes actifs. Comme indiqué ci-dessus, les composés utilisés sont préférentiellement des activateurs sélectifs de l'activité de l'AA4RP.

Comme indiqué ci-avant, le composé utilisé est préférentiellement un composé qui mime l'activité de l'AA4RP, stimule l'expression ou la sécrétion de l'AA4RP et/ou augmente la concentration de l'AA4RP dans les particules HDL qui sont réputées pour avoir un rôle protecteur contre l'athérosclérose, principalement du fait de leur capacité à extraire le cholestérol des cellules périphériques et à promouvoir son retour vers le foie où il est éliminé [27].

La présente divulgation concerne également des méthodes de traitement de pathologies cardiovasculaires comprenant l'administration à un sujet d'un activateur (sélectif) de l'AA4RP, notamment d'un composé qui mime l'activité de l'AA4RP, stimule l'expression ou la sécrétion de l'AA4RP et/ou augmente la concentration de l'AA4RP dans les particules HDL. L'administration peut être réalisée par toute voie classique pour ce type d'approche thérapeutique, comme notamment par voie systémique ou orale, en particulier, par injection, notamment intraveineuse, intradermique, sous-cutanée, intra-péritonéale, intramusculaire, intra-artérielle, etc..

### LEGENDE DES FIGURES

Figure 1 : Contrôle en SDS PAGE de la spécificité des anticorps anti-AA4RP et mise en évidence de la distribution de l'AA4RP dans les VLDL et les HDL.
Figure 2: Détection de l'AA4RP plasmatique par électrophorèse bidimensionnelle et blotting.
Figure 3 : Effet de la sur-expression de l'AA4RP sur le taux de triglycérides.
Figure 4 : Effet de la sur-expression de l'AA4RP sur le taux d'apo CIII dans des animaux transgéniques AA4RP et contrôles.
Figure 5 : Effet de la surexpression de l'AA4RP sur le taux d'apo CIII dans des animaux « Knock-Out » AA4RP homozygotes et hétérozygotes.
Figure 6 : Distribution des triglycérides au niveau des différentes lipoparticules chez des souris transgéniques AA4RP et contrôles.
Figure 7 : Activité enzymatique de la HL et de la LpL chez des souris transgéniques AA4RP humaine et contrôles.

### EXEMPLES

### Exemple 1 Séquence peptidique

### 1. Choix de la séquence peptidique appropriée

Le fragment peptidique suivant a été synthétisé. Ce fragment a été déterminé en utilisant différents algorithmes permettant de prédire la flexibilité, l'hydrophilicité, l'antigénicité, et les structures secondaires. Ce fragment de 95 acides aminés correspond aux résidus 20 à 114 de la séquence SEQ ID NO 1.

### 2. Synthèse peptidique.

Le peptide a été synthétisé par la méthode de synthèse en phase solide [21] sur un synthétiseur automatique modèle ABI 431 A (Applied Biosystems Inc., Californie, USA) en utilisant une stratégie Boc/Bzl sur une résine MBHA 0,5 mmol (0,57 mmol/g). Chaque acide aminé a été couplé deux fois en présence de dicyclohexylcarbodiimide/hydroxybenzotriazole sans capping. Les groupes protecteurs de chaîne latérale sont les suivants : Arg(Ts), Asp(Ochex), Glu(Ochex), Lys(2-C1-Z), His(Dnp), Ser(Bz1), Thr(Bzl), Met(O), Trp(formy) et Tyr(Br-Z) .

Le groupe Dnp sur le résidu Histidine a été éliminé du peptide avant clivage à partir du support par traitement en présence de β-mercaptoéthanol 10%, diisopropyléthylamine 5% dans un milieu DCM pendant 2 heures puis dans un milieu NMP pendant 2 heures. La résine peptidyle a été traitée avec du TFA 50% dans un milieu DCM pendant 20 minutes pour éliminer l'acide aminé terminal Boc. Le peptide a été clivé de la résine et déprotégé simultanément selon une procédure à HF lente et rapide : la résine (1 g) a été traitée avec du HF anhydre (2,5 ml) en présente de p-crésol (0,75 g), p-thiocrésol (0,25 g) et de diméthylsulfure (0,5 ml) à 0°C.

3 heures après, le fluorure d'hydrogène et le diméthylsulfure ont été éliminés par évaporation sous vide et les scavengers résiduels et les produits secondaires ont été extraits avec de l'éther diéthylique. Les appareils de la réaction ont été rechargés avec du p-crésol (0,75 g), du p-thiocrésol (0,25 g) et 10 ml de HF anhydre puis le mélange a été laissé en incubation à 0°C pendant 1 heure et demie. Le fluorure d'hydrogène a été éliminé par évaporation et le résidu a été mélangé en présence d'éther diéthylique. Le résidu a été filtré, lavé avec de l'éther diéthylique et extrait avec 200 ml d'une solution d'acide acétique aqueuse à 10% puis lyophilisé.

### 3. Spectrométrie de masse

La masse moléculaire a été déterminée en utilisant un spectromètre de masse à électrospray d'ions. Le spectre de l'électrospray a été obtenu en utilisant un appareil API (Perkin-Elmer-Sciex) sur un spectromètre de masse par électrospray d'ions à quadrupole simple, équipé avec un spray d'ions (électrospray assisté d'un nébuliseur) (Sciex, Toronto, Canada).

### 4. Immunisation

Le peptide a été émulsifié dans l'adjuvant complet de Freud et injecté par voie sous-cutanée à des lapins en utilisant 0,5 mg de peptide par injection pour les deux premières injections, suivi à 15 jours d'intervalle d'injections de rappel dans le même adjuvant mais en utilisant 0,25 mg de peptide seulement.

### Exemple 2 : Isolement d'anticorps de lapin anti-AA4RP spécifiques.

Les anticorps polyclonaux ont été isolés par précipitation avec 27% de sulfate de sodium puis purifiés par chromatographie d'affinité sur gel de sépharose 4B activé (pharmacia, Uppsala, Suède), couplé avec le peptide de l'AA4RP résidu 20 à 114 AA [28]. Les protéines non retenues sur le gel antigènique ont été éliminées par lavage avec une solution saline tamponnée phosphate (PBS : Phospate 50 mmol/L, pH 7,2, NaC1 150 mmol/L). Les fractions non liées de manière spécifique sur le gel AA4RP ont été éliminées par 25 mmol/L de PBS. L'élution des IgG polyclonales spécifiques de l'AA4RP a été accomplie en utilisant la glycine 0,2 M à pH 2,8. Les anticorps purifiés ont été dialysés immédiatement contre 10 mmol/L de PBS puis concentrés par ultrafiltration en utilisant un système Amicon (seuil de coupure 100 kD) (Amicon, Bervely, USA), testés en terme de contenu en protéine [29], puis conservés par aliquotes de 1 ml (1 mg) à - 30°C.

### 1. Analyse en Western Blot

### 1.1- Protocole:

La pureté et la spécificité de l'anticorps ont été testées en Western Blot [30].

Des particules VLDL, LDL et HDL humaines ont été soumises à une électrophorèse dénaturante sur gel SDS-PAGE (5 à 24%) puis transférées sur une membrane de nitrocellulose et mises à réagir avec l'anticorps anti-AA4RP humaine purifié. Les protéines immunoréactives ont été visualisées avec un anticorps polyclonal anti-IgG conjugué à la peroxydase de raifort (Sanofi-Diagnostics Pasteur, Mares-la-Coquette, France). Le développement de la réaction est effectué par chemiluminescence (Amersham, Pharmacia, Biotec).

### 1.2- Résultats:

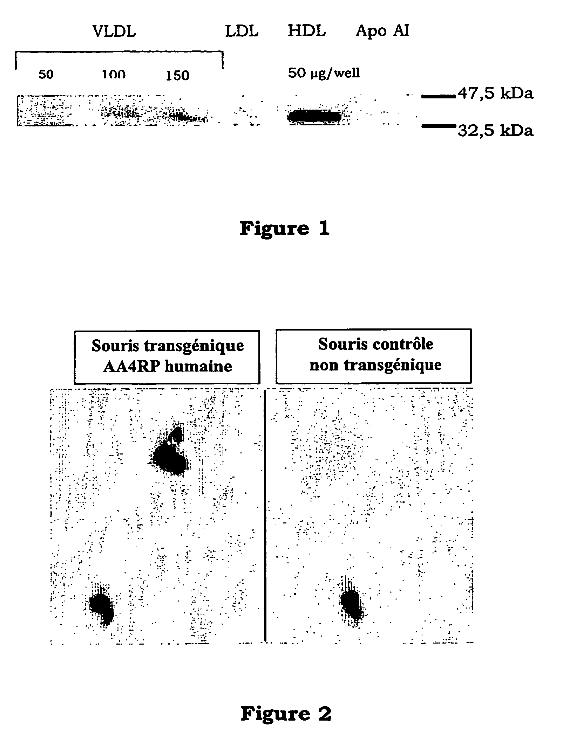

Les résultats obtenus sont présentés sur la Figure 1. Il apparaît sur cette figure que la bande spécifique qui est révélée par l'anticorps anti-AA4RP est située entre les marqueurs de poids moléculaire 32,5 et 47,5 kDa.

### 1.3- Interprétation :

Le résultat de l'immunoblot sur les différentes lipoprotéines humaines, montrent une localisation de l'AA4RP au niveau des VLDL et des HDL, avec une distribution très majoritaire au niveau des HDL.

La présence de l'AA4RP au niveau des VLDL expliquerait le rôle de cette apolipoprotéine dans la régulation du métabolisme de ces lipoprotéines riche en triglycérides, et donc la modulation de la concentration de ces lipides athérogènes.

La localisation de l'AA4RP au niveau des HDL est certainement en relation avec le rôle de ces particules dans le transport inverse du cholestérol. En effet la plupart des apolipoprotéines localisées au niveau des HDL sont des promoteurs de la captation du cholestérol des cellules périphériques par les HDL et de son retour vers le foie où il est éliminé.

### 2. Electrophorèse bidimensionnelle et blotting

### 2.1- Protocole:

Les échantillons étudiés sont :
- un plasma de souris contrôle,
- un plasma de souris transgénique AA4RP humaine.

20 µl de chaque échantillon ont été appliqués dans des puits d'échantillons de 1,5 cm dans un gel d'agarose à 0,75% dans un tampon barbital 50 mmol à pH 8,6 sur Gelbond (FMC Bio-Products, New Jersey, USA). L'électrophorèse a été réalisée dans la première dimension jusqu'à ce que le marqueur albumine coloré par le bleu de bromophénol ait migré de 7,5 cm. L'électrophorèse dans la seconde dimension a été réalisée dans un gradient de gel de polyacrylamide de 2 à 15% (15 x 15 cm). Des échantillons (0,5 x 7 cm) ont été découpés du gel d'agarose, placés de manière horizontale les uns à la suite des autres dans le gel de polyacrylamide et recouverts par de l'agarose à 1%. L'électrophorèse a été réalisée à 4°C dans 25 mM de tris-(hydroxyméthyl) aminomethane (Tris)-glycine (pH 8,3) à 100 V pendant 19 heures. Les gels ont été immunoblottés sur des membranes de nitrocellulose (Sartorius 0,45 µm) dans un tampon de 25 mM tris- (hydroxyméthyl) aminomethane (Tris)-glycine (pH 8,3) en utilisant un système de blotting semi-sec (Pharmacia, umeå, Suède). Les membranes ont été incubées avec les anticorps polyclonaux anti- et ceux liés spécifiquement à l'AA4RP sont révélés par un anti IgG de lapin marqué à la peroxydase. Le développement de la réaction enzymatique est effectué par chemiluminescence (Amersham, Pharmacia, Biotec).

### 1.2- Résultats :

Les résultats obtenus sont présentés sur la Figure 2. Ils montrent que, chez les souris transgéniques ou contrôle, on observe que l'AA4RP est située au niveau des préβ-HDL, alors que la présence de l'apolipprotéine au niveau des α-HDL n'est observée que dans le cas des souris transgéniques AA4RP humaine.

### 1.3- Interprétation :

La présence de l'AA4RP au niveau des préβ-HDL est un deuxième argument en faveur de l'implication de cette apolipoprotréine dans l'efflux du cholestérol des cellules périphériques vers le foie. La présence de façon importante de l'AA4RP au niveau des α-HDL après transgénèse, alors qu'elle est totalement absente dans cette sous fraction d'HDL chez les contrôles, est un élément très intéressant. Cette redistribution différente serait en liaison avec la maturation des HDL, où l'AA4RP modulerait les différents facteur impliqués dans le métabolisme des HDL, tel que la CETP (Cholesteryl Ester Transfer Protein), la LCAT (Lecithin: Cholesterol AcylTransferase) ou la PLTP (Phospholipide Transfer Protein).

### Exemple 3 : Effet de la sur-expression d'AA4RP sur le taux de triglycérides

### 1.- Protocole:

Des échantillons provenant de souris transgéniques AA4RP ou de souris contrôles, ayant subit un régime hyperlipidique, ont été dilués au 1/3 ou au 1/5 dans du sérum physiologique, puis dosés en triglycérides contre une gamme standard, préparée grâce au calibrateur des lipides CFAS Réf. N° 759350 (Boehringer Mannheim GmbH, Allemagne). La gamme standard a été construite de 16 à 500 µg/ml. 100 µl de chaque dilution d'échantillon ou de gamme étalon sont déposés par puits d'une plaque de titration (96 puits). Ensuite 200µl de réactifs triglycérides Réf. 701912 (Boehringer Mannheim GmbH, Allemagne) sont rajoutés dans chaque puits, et l'ensemble de la plaque est incubé pendant 30 min. à 37°C. La lecture des Densités Optiques (DO) est effectuée à 492 nm sur le spectrophotomètre. Les concentrations en triglycérides de chaque échantillon sont calculées après construction de la courbe étalon selon une fonction linéaire y=ax+b, où y représente les DO et x les concentrations en triglycérides.

### 2- Résultats :

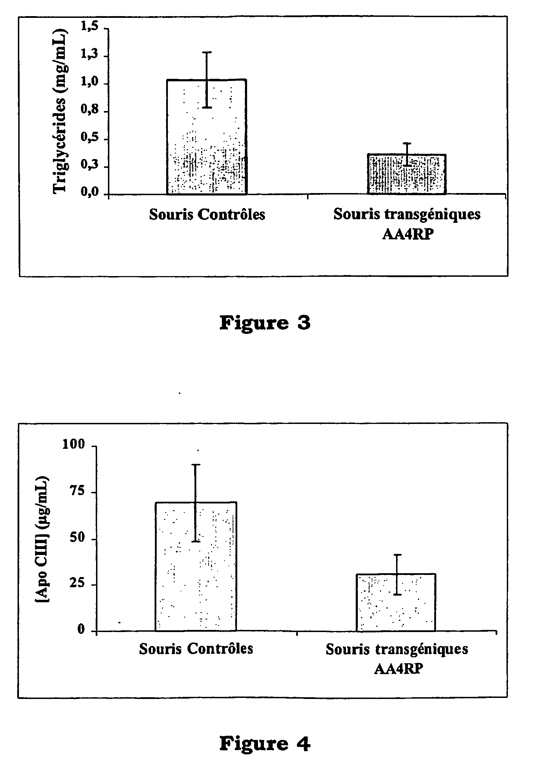

Les résultats obtenus sont présentés dans le Tableau I ci-dessous ainsi que dans la Figure 3.

**Tableau I**

| | Souris Contrôles | Souris transgéniques AA4RP |
|---|---|---|
| Nombre de sujets | 13 | 21 |
| Triglycérides (mg/ml) | 1,039 | 0,361 |
| Ecart type (mg/ml) | 0,249 | 0,101 |

Les résultats obtenus montrent que la diminution des triglycérides est très significative (test de student p<0,001) chez les souris transgéniques AA4RP.

### 3- Interprétation :

L'augmentation des triglycérides chez les souris contrôle après un régime riche en lipides est fortement atténuée après transgénèse. Cette baisse est probablement due à une accélération du catabolisme des lipoprotéines riches en triglycérides tel que les chylomicrons et les VLDL, suite à une augmentation de l'activité des différents acteurs de ce catabolisme, comme par exemple une augmentation de l'activité de la lipoprotéine lipase (LpL), une augmentation des cofacteurs de l'activation de la LpL ou une baisse de facteur inhibant cette activité comme l'apo CIII.

### Exemple 4 : Effet de la sur-expression d'AA4RP sur le taux d'Apo CIII

### 1- Protocole :

L'apo CIII des souris sur-exprimant l'AA4RP humaine a été dosée par la méthode immuno-néphélométrique sur le système d'immunochimie ARRAY® (Beckman Coulter, Villepinte, France). Un pool de sérum de souris normolipidiques a été utilisé comme standard dont la valeur en apo CIII est de 25 µg/ml. Une gamme étalon allant de 4 µg/ml à 25 µg/ml, est construite par dilution du standard dans du phosphate potassique 0,01 M pH 7,2.

Les échantillons de plasma de souris sur-exprimant l'AA4RP humaine ou non, ayant obtenues un régime hyperlipidique, et des échantillons de plasma de souris homozygotes knock out (-/-), hétérozygotes (+/-) ou de type sauvage (+/+) sont dilués au 1/6 ou au 1/12 dans les même conditions que le standard. 42 µl de chaque point de la gamme étalon ainsi que chaque dilution d'échantillon des différentes souris impliquées dans l'étude, sont mis en contact avec 42 µl d'anti-apo CIII de souris (dans une cuve réactionnelle contenant du polyéthylèneglycol (PEG)). La concentration en apo CIII de l'échantillon est directement proportionnelle au taux de lumière réfléchie par le précipitât formé lors de la réaction anticorps-antigène

### 2- Résultats :

### a-) Apo CIII des souris transgéniques AA4RP et des souris contrôles (régime riche en lipides)

Les résultats obtenus sont présentés dans le Tableau II ci-dessous ainsi que dans la Figure 4.

**Tableau II :**

| | Souris contrôles | Souris Transgéniques AA4RP |
|---|---|---|
| Nombre de sujets | 9 | 20 |
| Apo CIII (µg/ml) | 69,29 | 30,70 |
| Ecart type (µg/ml) | 20,90 | 10,88 |

Les résultats obtenus montrent que l'apo CIII est diminuée d'environ trois fois chez les souris transgéniques AA4RP.

### b-) Apo CIII des souris knock out AA4RP homozygotes (-/-), hétérozygotes (+/-) et de type sauyage(+/-) :

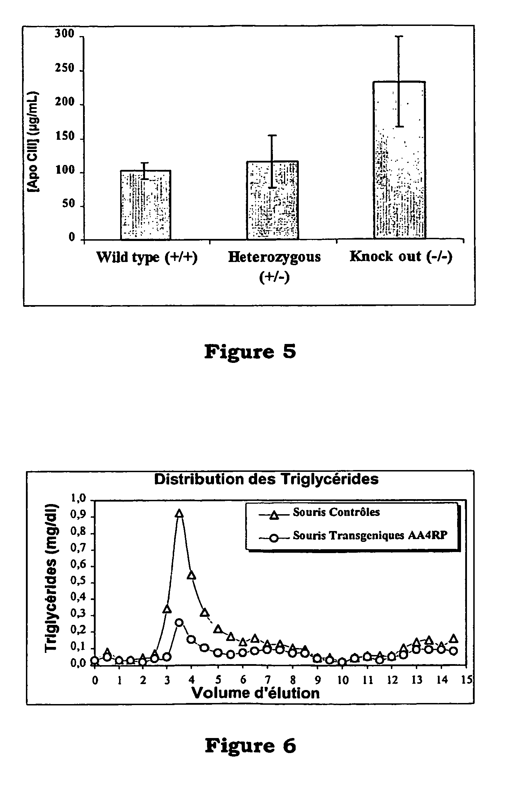

Les résultats obtenus sont présentés dans le Tableau III ci-dessous ainsi que dans la Figure 5.

**Tableau III :**

| | Type sauvage (+/+) | Souris KO (+/-) | Souris KO (-/-) |
|---|---|---|---|
| Nombre | 6 | 11 | 6 |
| Apo CIII (µg/ml) | 102,58 | 115,83 | 231,75 |
| Ecart type (µg/ml) | 11,94 | 38,24 | 66,33 |

Les résultats obtenus montrent que les souris knock out AA4RP présentent les concentrations les plus élevées de l'apo CIII.

### 3- Interprétation :

Les résultats de la baisse de l'apo CIII chez les souris transgéniques AA4RP humaine après un régime riche en lipides ou les résultats de l'augmentation de l'apo CIII chez les souris knock out AA4RP, vont en parallèle avec les résultats observées lors de la détermination des concentrations des triglycérides, confirmant ainsi le rôle positif de l'AA4RP dans l'élimination des particules remnants (résiduelles) riche en triglycérides.

### Exemple 5 : Distribution des triglycérides au niveau des différentes lipoprotéines

### 1- Protocole :

La distribution des triglycérides au niveau des VLDL, LDL et HDL est étudiée par fractionnement de 200 µl de plasma de souris transgénique AA4RP ou souris contrôle après un régime hyperlipidique.

Le fractionnement des déférentes particules est effectué sur une colonne superose 6 de Pharmacia intégrée dans un système AKTA FPLC (Pharmacia Biotech, umeå, Suède). Les différentes fractions obtenues sont éluées par un tampon PBS 0,01 M, NaCl 0,1 M, pH 7,2 avec un débit de 0,2 ml par minutes. La concentration des triglycérides au niveau de chaque fraction est déterminée comme précédemment.

### 2- Résultats:

Les résultats obtenus sont présentés sur la Figure 6.

### 3- Interprétation :

L'accumulation des particules riche en triglycérides chez les souris contrôles après un régime riche en lipides, n'est pas observée chez les souris transgéniques AA4RP, ce qui confirme le rôle de l'AA4RP dans l'élimination des particules remnants (résiduelles).

### Exemple 6 : l'activité de la LpL et de la HL dans les souris transgèniques AA4RP humaine.

### 1- Protocole :

Le test est effectué sur les plasmas post-héparine (prélevés 5 minutes après injection de l'héparine) en provenance de souris transgéniques et de souris contrôles à fond génétique FVB/N. L'activité lypasique totale a été déterminée par incubation avec de la trioléine radioactive et en présence d'un co-facteur (l'Apo CII). L'activité HL a été déterminée après incubation de chaque plasma avec la trioléine mais cette fois ci en l'absence de l'Apo CII et en présence de 1 M de NaCl pour éliminer l'activité de la LpL. L'activité lipasique de chaque plasma est ensuite estimée en dosant le nombre de moles d'acide gras libérés. Enfin l'activité LpL est déduite par soustraction de l'activité HL de l'activité lypasique totale.

### 2- Résultats:

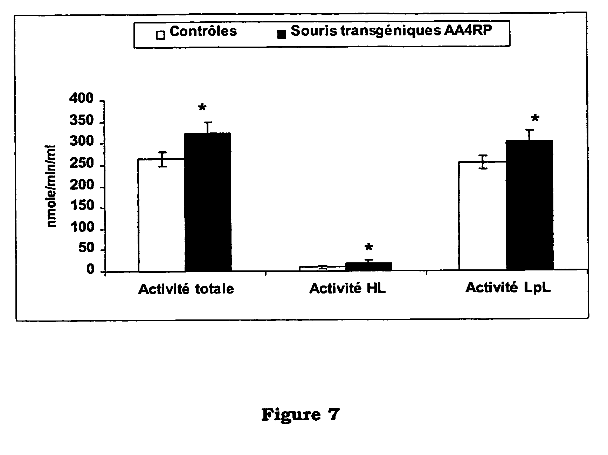

Les résultats obtenus sont présentés sur la Figure 7 qui montre une augmentation significative de l'activité lypasique totale de l'activité HL et de l'activité LpL chez les souris transgéniques AA4RP par rapport aux souris contrôles.

### 3- Interprétation :

L'augmentation de l'activité enzymatique de la LpL et de la HL chez les souris transgéniques AA4RP est en concordance avec l'activité de l'AA4RP :
- diminution des taux de triglycérides et d'Apo CIII (observée dans les exemples 3, 4 et 5)
- augmentation de l'efflux de cholestérol et localisation de l'AA4RP dans les HDL et les préβ-HDL afin de participer à leur métabolisme (exemple 2).

### REFERENCES BIBLIOGRAPHIQUES

1. Brewer, H.B., Shulman R., Herbert P., Roman R. and Werrly K., The complete amino acids sequence of alanine apolipoprotein from plasma very low density lipoprteins. J. Biol. Chem., 1974. 249: p. 4975-4984.
2. Lenich, C., Brecher P., Makrides S., Chobanian A. and Zannis V.I., Apolipoprotein gene expression in rabbit: abundance, size and distribution of apolipoprotein mRNA species in different tissues. J. Lip. Res., 1988. 29: p. 755-764.
3. Hoddis, H.N., and Mack W.J., Triglyceride-rich lipoproteins and the progression of coronary artery disease. Curr. Opin. Lipidol., 1995. 6: p. 209-214.
4. Sacks, F.M., Alaupovic P., Moye L. A., Cole T. G., Sussex B., Stampfer M. J., Pfeffer M. A. and Braunwald E., VLDL, apolipoproteins B, CIII, and E, and risk of recurrent coronary events in the Cholesterol and Recurrent Events (CARE) trial. Circulation, 2000. 102(16): p. 1886-92.
5. Kuusi, T., P.K. Kinnunen, and E.A. Nikkila, Hepatic endothelial lipase antiserum influences rat plasma low and high density lipoproteins in vivo. FEBS Lett, 1979. 104(2): p. 384-8.
6. Jansen, H., et al., On the dual localization of lipoprotein lipase in rat heart. Studies with a modified perfusion technique. Biochem Biophys Res Commun, 1980. 92(2): p. 411-6.
7. Shirai, K., R.L. Barnhart, and R.L. Jackson, Hydrolysis of human plasma high density lipoprotein 2- phospholipids and triglycerides by hepatic lipase. Biochem Biophys Res Commun, 1981. 100(2): p. 591-9.
8. Castro, G.R. and C.J. Fielding, Early incorporation of cell-derived cholesterol into pre-beta-migrating high-density lipoprotein. Biochemistry, 1988. 27(1): p. 25-9.
9. Huang, Y., A. von Eckardstein, and G. Assmann, Cell-derived unesterified cholesterol cycles between different HDLs and LDL for its effective esterification in plasma. Arterioscler Thromb, 1993. 13(3): p. 445-58.
10. Francone, O.L., A. Gurakar, and C. Fielding, Distribution and functions of lecithin:cholesterol acyltransferase and cholesteryl ester transfer protein in plasma lipoproteins. Evidence for a functional unit containing these activities together with apolipoproteins A-I and D that catalyzes the esterification and transfer of cell-derived cholesterol. J Biol Chem, 1989. 264(12): p. 7066-72.
11. Barrans, A., et al., Hepatic lipase induces the formation of pre-beta 1 high density lipoprotein (HDL) from triacylglycerol-rich HDL2. A study comparing liver perfusion to in vitro incubation with lipases. J Biol Chem, 1994. 269(15): p. 11572-7.
12. Westhof, E., et al., Correlation between segmental mobility and the location of antigenic determinants in proteins. Nature, 1984. 311(5982): p. 123-6.
13. Karplus, P.A., and G. E. Schulz, Prediction of chain flexibility in proteins. Naturwiss-enschaften, 1985. 72: p. 212-216.
14. Hoop, T., and K. Woods, A computer program for predicting protein antigenic determinants. Molecular Immunology, 1983. 20: p. 483-489.
15. Kyte, J. and R.F. Doolittle, A simple method for displaying the hydropathic character of a protein. J Mol Biol, 1982. 157(1): p. 105-32.
16. Jameson, B.A., and H. Wolf, The antigenic index : a novel algorithm for predicting antigenic determinants. Comput. Appl. Biosci., 1988. 4: p. 181-186.
17. Chou, P.Y. and G.D. Fasman, Prediction of the secondary structure of proteins from their amino acid sequence. Adv Enzymol Relat Areas Mol Biol, 1978. 47: p. 45-148.
18. Garnier, J., D.J. Osguthorpe, and B. Robson, Analysis of the accuracy and implications of simple methods for predicting the secondary structure of globularproteins. J Mol Biol, 1978. 120(1): p. 97-120.
19. Vaitukaitis, J., et al., A method for producing specific antisera with small doses of immunogen. J Clin Endocrinol Metab, 1971. 33(6): p. 988-91.
20. Bassiri, R.M., Dvorak J. and Utiger R.D., Thyrotrpin-releasing hormone, in Methods of hormone radioimmunoassay, B.M. In: Jaffe, and Behrman H.R. (Eds), Editor. 1979, New York Academic Press: New York. p. p: 46.
21. Merrifield, R.B., Solide phase peptide synthesis. The synthesis of a tetrapeptide. J. Am. Chem. Soc, 1963. 85: p. 2149-2154.
22. Harlow, E.a.L., D., ed. Antibodies: a Laboratory Manual. 1988, Cold Spring Harbor Laboratory Publications: New York. pp: 139-243.
23. Kohler, G. and C. Milstein, Continuous cultures of fused cells secreting antibody of predefined specificity. Nature, 1975. 256(5517): p. 495-7.
24. Ward, E.S., et al., Binding activities of a repertoire of single immunoglobulin variable domains secreted from Escherichia coli. Nature, 1989. 341(6242): p. 544-6.
25. Jones, P.T., Dear P. H., Foote J., Neuberger M. S. and Winter G., Replacing the complementarity-determining regions in a human antibody with those from a mouse. Nature, 1986. 321: p. 522-525.
26. Riechmann, L., Clark M., Waldmann H. and Winter G., Monoclonal antibody therapeutic trials in seven patients with T-cell lymphoma. Nature, 1988. 332: p. 323-327.
27. Fruchart, J.C. and P. Duriez, High density lipoproteins and coronary heart disease. Future prospects in gene therapy. Biochimie, 1998. 80(2): p. 167-72.
28. Axen, R., J. Porath, and S. Ernback, Chemical coupling of peptides and proteins to polysaccharides by means of cyanogen halides. Nature, 1967. 214(95): p. 1302-4.
29. Lowry O.H., R.J., Farr A.L., and Randall R.J., Protein measurement with folin phenol reagent. J. Biol. Chem., 1951. 193: p. 265-275.
30. Towbin, H., T. Staehelin, and J. Gordon, Electrophoretic transfer of proteins from polyacrylamide gels to nitrocellulose sheets: procedure and some applications. Proc Natl Acad Sci U S A, 1979. 76(9): p. 4350-4.

## Revendications

1. Procédé pour la sélection, l'identification ou la caractérisation de composés permettant de réduire le taux d'apolipoprotéine CIII circulante ou de lipoparticules riches en apolipoprotéine CIII et/ou d'augmenter l'activité de la lipoprotéine lipase et/ou de la lipase hépatique et/ou d'augmenter le transport inverse du cholestérol chez un mammifère, comprenant la détermination de la capacité d'un composé test à augmenter l'activité de la protéine AA4RP et la sélection, l'identification ou la caractérisation de composés ainsi testés permettant de réduire le taux d'apolipoprotéine CIII circulante ou de lipoparticules riches en apolipoprotéine CIII et/ou d'augmenter l'activité de la lipoprotéine lipase et/ou de la lipase hépatique et/ou d'augmenter le transport inverse du cholestérol chez un mammifère.

2. Procédé selon la revendication 1, **caractérisé en ce qu'**il comprend la détermination in vitro ou ex vivo de la capacité d'un composé test à augmenter la synthèse de la protéine AA4RP.

3. Procédé selon la revendication 1 ou 2, **caractérisé en ce qu'**il comprend la mise en contact in vitro ou ex vivo d'un composé test avec une cellule comprenant un gène rapporteur sous le contrôle d'un promoteur transcriptionnel comprenant tout ou une partie de la séquence du promoteur du gène AA4RP, et la détermination de la capacité dudit composé test à augmenter l'expression du gène rapporteur.

4. Procédé selon la revendication 1, **caractérisé en ce qu'**il comprend la détermination de la capacité d'un composé test à augmenter l'incorporation de la protéine AA4RP dans les particules HDL ou VLDL, en particulier dans les particules HDL.

5. Polypeptide, **caractérisé en ce qu'**il consiste en la séquence des résidus 20-114 de la séquence SEQ ID NO 1.

6. Composition, **caractérisée en ce qu'**elle comprend un polypeptide défini selon la revendication 5.

7. Composition pharmaceutique, **caractérisée en ce qu'**elle comprend un polypeptide défini selon la revendication 5 et un véhicule acceptable sur le plan pharmaceutique.

8. Composition pharmaceutique selon la revendication précédente, destinée à réguler, en particulier augmenter ou mimer, l'activité de l'AA4RP *in vivo.*

9. Composition pharmaceutique selon la revendication 7 ou 8, destinée au traitement curatif ou préventif de pathologies cardiovasculaires ou du syndrome métabolique par diminution du taux d'apolipoprotéine CIII dans le sang d'un patient, en particulier de lipoparticules riches en apolipoprotéine CIII, ou par augmentation de l'activité de la LpL et/ou de la HL ou par augmentation du transport inverse du cholestérol.

## Claims

1. A method for selecting, identifying or characterizing compounds for reducing the level of circulating apolipoprotein CIII or lipoparticles rich in apolipoprotein CIII and/or increasing the activity of lipoprotein lipase and/or of hepatic lipase and/or increasing reverse cholesterol transport in a mammal, comprising determining the ability of a test compound to increase the activity of the AA4RP protein and the selection, identification or characterisation of compounds thus tested for reducing the level of circulating apolipoprotein CIII or lipoparticles rich in apolipoprotein CIII and/or increasing the activity of lipoprotein lipase and/or of hepatic lipase and/or increasing reverse cholesterol transport in a mammal.

2. A method according to claim 1, wherein it comprises determining *in vitro* or *ex vivo* the ability of a test compound to increase the synthesis of the AA4RP protein.

3. A method according to claim 1 or 2, wherein it comprises contacting *in vitro* or *ex vivo* a test compound with a cell comprising a reporter gene under the control of a transcriptional promoter containing all or part of the sequence of the AA4RP gene promoter, and determining the ability of said test compound to increase the expression of the reporter gene.

4. A method according to claim 1, wherein it comprises determining the ability of a test compound to increase the incorporation of the AA4RP protein into HDL or VLDL particles, in particular in HDL particles.

5. Polypeptide, **characterized in that** it consists of 20-114 of the sequence SEQ ID NO 1.

6. A composition, wherein it comprises a polypeptide as defined in claim 5.

7. A pharmaceutical composition, wherein it comprises a polypeptide as defined in claim 5 and a pharmaceutically acceptable vehicle.

8. The pharmaceutical composition according to the preceding claim, for regulating, in particular increasing or mimicking, AA4RP activity *in vivo.*

9. The pharmaceutical composition according to claim 7 or 8, for the curative or preventive treatment of cardiovascular pathologies or metabolic syndrome by reducing apolipoprotein CIII levels in the blood of a patient, in particular lipoproteins rich in apolipoprotein CIII, or by increasing the activity of LpL and/or of HL or by increasing reverse cholesterol transport.

## Patentansprüche

1. Verfahren zur Selektion, Identifikation oder Charakterisierung von Verbindungen, die es ermöglichen, bei einem Säuger den Spiegel von zirkulierendem Apolipoprotein CIII oder von Apolipoprotein-CIII-reichen Lipoproteinpartikeln zu reduzieren und/oder die Aktivität der Lipoproteinlipase und/oder der hepatischen Lipase zu erhöhen und/oder den reversen Cholesteroltransport zu erhöhen, umfassend die Bestimmung der Fähigkeit einer Testverbindung, die Aktivität des Proteins AA4RP zu erhöhen, und die Selektion, die Identifizierung oder die Charakterisierung so getesteter Verbindungen, die es ermöglichen, bei einem Säuger den Spiegel von zirkulierendem Apolipoprotein CIII oder von Apolipoprotein-CIII-reichen Lipoproteinpartikeln zu reduzieren und/oder die Aktivität der Lipoproteinlipase und/oder der hepatischen Lipase zu erhöhen und/oder den reversen Cholesteroltransport zu erhöhen.

2. Verfahren nach Anspruch 1, **dadurch gekennzeichnet, dass** es die in vitro- oder ex vivo-Bestimmung der Fähigkeit einer Testverbindung, die Synthese des Proteins AA4RP zu erhöhen, umfasst.

3. Verfahren nach Anspruch 1 oder 2, **dadurch gekennzeichnet, dass** es das in vitro- oder ex vivo-Inkontaktbringen einer Testverbindung mit einer Zelle umfasst, die ein Reportergen unter der Kontrolle eines Transkriptionspromotors umfasst, der die Gesamtheit oder einen Teil der Sequenz des Promotors des AA4RP-Gens umfasst, sowie die Bestimmung der Fähigkeit der Testverbindung, die Expression des Reportergens zu erhöhen.

4. Verfahren nach Anspruch 1, **dadurch gekennzeichnet, dass** es die Bestimmung der Fähigkeit einer Testverbindung umfasst, die Einfügung des Proteins AA4RP in die HDL- oder VLDL-Partikel, insbesondere in die HDL-Partikel, zu erhöhen.

5. Polypeptid, **dadurch gekennzeichnet, dass** es aus der Sequenz der Reste 20-114 der Sequenz SEQ ID NO:1 besteht.

6. Verbindung, **dadurch gekennzeichnet, dass** sie ein nach Anspruch 5 definiertes Polypeptid umfasst.

7. Arzneimittel, **dadurch gekennzeichnet, dass** es ein nach Anspruch 5 definiertes Polypeptid und einen pharmazeutisch verträglichen Träger umfasst.

8. Arzneimittel nach vorstehendem Anspruch, das dazu bestimmt ist, die Aktivität von AA4RP *in vivo* zu regulieren, insbesondere zu erhöhen oder nachzuahmen.

9. Arzneimittel nach Anspruch 7 oder 8, das bestimmt ist zur heilenden oder vorbeugenden Behandlung von Herz- und Gefäßkrankheiten oder des metabolischen Syndroms durch Senkung des Apolipoprotein-CIII-Spiegels im Blut eines Patienten, insbesondere des Spiegels der Apolipoprotein-CIII-reichen Lipoproteinpartikel, oder durch Erhöhung der Aktivität der LpL und/oder der HL oder durch Erhöhung des reversen Cholesteroltransports.


### LISTE DE SEQUENCES

<110> GENFIT SA
<120> Methodes de criblage de molecules utiles pour la prévention ou le traitement du syndrome metabolique, des maladies cardiovasculaires et de l'atherosclerose
<130> B0093WO
<140>
   <141>
<160> 2
<170> PatentIn Ver. 2.1
<210> 1
   <211> 366
   <212> PRT
   <213> Homo sapiens
<400> 1
   Met Ala Ser Met Ala Ala Val Leu Thr Trp Ala Leu Ala Leu Leu Ser
   1               5                   10                  15
   Ala Phe Ser Ala Thr Gln Ala Arg Lys Gly Phe Trp Asp Tyr Phe Ser
               20                  25                  30
   Gln Thr Ser Gly Asp Lys Gly Arg Val Glu Gln Ile His Gln Gln Lys
           35                  40                  45
   Met Ala Arg Glu Pro Ala Thr Leu Lys Asp Ser Leu Glu Gln Asp Leu
       50                  55                  60
   Asn Asn Met Asn Lys Phe Leu Glu Lys Leu Arg Pro Leu Ser Gly Ser
   65                  70                  75                  80
   Glu Ala Pro Arg Leu Pro Gln Asp Pro Val Gly Met Arg Arg Gln Leu
                   85                  90                  95
   Gln Glu Glu Leu Glu Glu Val Lys Ala Arg Leu Gln Pro Tyr Met Ala
               100                 105                 110
   Glu Ala His Glu Leu Val Gly Trp Asn Leu Glu Gly Leu Arg Gln Gln
           115                 120                 125
   Leu Lys Pro Tyr Thr Met Asp Leu Met Glu Gln Val Ala Leu Arg Val
   130 135 140
   Gln Glu Leu Gln Glu Gln Leu Arg Val Val Gly Glu Asp Thr Lys Ala
   145 150 155 160
   Gln Leu Leu Gly Gly Val Asp Glu Ala Trp Ala Leu Leu Gln Gly Leu
   165 170 175
   Gln Ser Arg Val Val His His Thr Gly Arg Phe Lys Glu Leu Phe His
   180 185 190
   Pro Tyr Ala Glu Ser Leu Val Ser Gly Ile Gly Arg His Val Gln Glu
   195 200 205
   Leu His Arg Ser Val Ala Pro His Ala Pro Ala Ser Pro Ala Arg Leu
   210 215 220
   Ser Arg Cys Val Gln Val Leu Ser Arg Lys Leu Thr Leu Lys Ala Lys
   225 230 235 240
   Ala Leu His Ala Arg Ile Gln Gln Asn Leu Asp Gln Leu Arg Glu Glu
   245 250 255
   Leu Ser Arg Ala Phe Ala Gly Thr Gly Thr Glu Glu Gly Ala Gly Pro
   260 265 270
   Asp Pro Gln Met Leu Ser Glu Glu Val Arg Gln Arg Leu Gln Ala Phe
   275 280 285
   Arg Gln Asp Thr Tyr Leu Gln Ile Ala Ala Phe Thr Arg Ala Ile Asp
   290 295 300
   Gln Glu Thr Glu Glu Val Gln Gln Gln Leu Ala Pro Pro Pro Pro Gly
   305 310 315 320
   His Ser Ala Phe Ala Pro Glu Phe Gln Gln Thr Asp Ser Gly Lys Val
   325 330 335
   Leu Ser Lys Leu Gln Ala Arg Leu Asp Asp Leu Trp Glu Asp Ile Thr
   340 345 350
   His Ser Leu His Asp Gln Gly His Ser His Leu Gly Asp Pro
   355 360 365
<210> 2
   <211> 1060
   <212> ADN
   <213> Séquence artificielle
<220>
   <223> Description de la séquence artificielle: Promoteur AA4RP
<400> 2
   atgtcccttg cagccaagtc taattctaac taatacaaac taactctaaa aaatgaatat 60
   atattcacca ggggataggc tatttcaagc agagggaagc ctgtataaag gctcagggaa 120
   tgctgtggtt ttatgtggca gcagatgaga ctggaaatga gtcaggatga gccacagtgg 180
   aggatgaatt aaatgggcag gagtgtggta gaaagacctg ttggaggcta tgaatgcaat 240
   caaggtgaca gacaactggt gcaatgatgg tagtggaaat ggaggagagg ggattgattc 300
   aagatgcatt taggaccaag aatcgggagc ttgtgaacgt gtgtatgagt actgtagacg 360
   gagtgggtgt gtcatcagag aagatctgag catttgggct tgctctcctc agaggccctg 420
   cgagtggagt tcagcttttc ctcatggggc aaatctyact ttcgctccag ttcctggggc 480
   tcagagtccc tggcccagat gcctcttgcc atctcatctt caccctgcct ggcttccctt 540
   gcttgttcca ggattgtttc ataaagaggg atgtggttgg tctttaaccc tatgaatgct 600
   ggctgaggat gcctgcggaa cctgtagtga agctttcagg ggctgctcgg gttctggctg 660
   gtaggtgaac actgtccatc ttgccggctg ggacacagtg actctgggta gttgtgtaag 720
   agaggggccc ttggcagaca aacaggttct tctctgttgg tgggccagcc agcaggtcag 780
   tgggaaggtt aaaggtcatg gggtttggga gaaactgggt gaggagttca gccccatccc 840
   ccgtaaagct cctgggaagc acttctctac tggggcagcc cctgatacca gggcactcat 900
   taaccctctg ggtgccaggg aaagggcagg aggtgagtgc tgggaggcag ctgaggtcaa 960
   cttcttttga acttccacgt ggtatttact cagagcaatt ggtgccagag gctcagggcc 1020
   ctggagtata aagcagaatg tctgctctct gtgcccagac                       1060